Europäisches Patentamt

European Patent Office

Office européen des brevets

(19)

(11) Veröffentlichungsnummer: **0 043 128**
**B1**

(12) **EUROPÄISCHE PATENTSCHRIFT**

(45) Veröffentlichungstag der Patentschrift:
23.11.83

(51) Int. Cl.³: **A 61 K 7/48**

(21) Anmeldenummer: **81104991.5**

(22) Anmeldetag: **26.06.81**

(54) **Kosmetische Mittel.**

(30) Priorität: **27.06.80 DE 3024318**

(43) Veröffentlichungstag der Anmeldung:
**06.01.82 Patentblatt 82/1**

(45) Bekanntmachung des Hinweises auf die Patenterteilung:
**23.11.83 Patentblatt 83/47**

(84) Benannte Vertragsstaaten:
**AT CH DE FR GB IT LI**

(56) Entgegenhaltungen:
**DE - A - 2 617 919**
**FR - A - 1 096 421**
**FR - A - 1 112 301**
**US - A - 4 057 627**

**CHEMICAL ABSTRACTS, Band 87, Nr. 18, veröffentlicht am 31. Oktober 1977, Seite 315, Zusammenfassung 141124b COLUMBUS OHIO (US)**
**CHEMICAL ABSTRACTS, Band 88, Nr. 16, veröffentlicht am 17. April 1978, Seite 334, Zusammenfassung 110537e COLUMBUS, OHIO (US)**

(73) Patentinhaber: **Chemisches Laboratorium Dr. Kurt Richter GmbH, Bennigsenstrasse 25, D-1000 Berlin 41 (DE)**

(72) Erfinder: **Kludas, Martin, Dr.med., Herthastrasse 22, D-1000 Berlin 33 (DE)**
Erfinder: **Heise, Dieter, Dr. rer.nat., Tristanstrasse 2a, D-1000 Berlin 39 (DE)**

(74) Vertreter: **Mitscherlich, Hans, Dipl.-Ing. Patentanwälte Dipl.-Ing.H. Mitscherlich et al, Dipl.-Ing. K. Gunschmann Dr.rer. nat. W. Körber Dipl.-Ing. J. Schmidt-Evers Steinsdorfstrasse 10, D-8000 München 22 (DE)**

EP 0 043 128 B1

## Kosmetische Mittel

Es ist bekannt, dass übermässige Sonnenbestrahlung chronische Hautschäden bewirken kann, die zu epidermalen Veränderungen, wie Atrophie, Pigmentverschiebungen, kollagener Degeneration bis hin zu bleibenden Schäden an der Desoxyribonucleinsäure (DNS) der Zellen führen können. Die Reihenfolge der Bausteine der doppelstrangigen Helixstruktur der DNS gewährleistet bekanntlich bei der Zellteilung die richtige Wiedergabe der genetisch codierten Erbanlagen. Intensive Einwirkung von UV-Strahlen auf die Haut führt zwischen zwei benachbarten Thymidinbausteinen der DNS durch Cycloaddition zu einer Thymidindimerisierung. Diese Strukturänderung der Nucleinsäure kann entweder zur Abtötung der Zelle oder zu einem vererbbaren Zellschaden führen.

Jede Zelle besitzt jedoch ein sogenanntes endogenes Repair-System, bestehend aus einer enzymatisch gestreuten Reaktionsfolge, um die Thymidindimeren aus dem DNS-Strang herauszuschneiden und durch ein Thymidinmonomer zu ersetzen.

Dieser natürliche DNS-Reparaturmechanismus besitzt allerdings nur eine begrenzte Kapazität, die sich durch eine übermässig starke Einwirkung von Licht und Sonne weitgehend erschöpft. Ausserdem ist aus Untersuchungen (J.B. Little „Gerontology" 22, 28-55 [1976]) bekannt, dass zwischen der Alterung einer Zelle und ihrer Fähigkeit, einen normalen Reparaturprozess ablaufen zu lassen, ein enger Zusammenhang besteht, wobei noch zu klären bleibt, ob ein Nachlassen der Reparaturfähigkeit der Zelle die direkte Ursache für die Alterung ist oder nur einen beschleunigenden Faktor darstellt.

Der vorliegenden Erfindung liegt nun die Aufgabe zugrunde, Mittel zur Verfügung zu stellen, deren Anwendung es ermöglicht, die erwähnte, durch übermässigen Lichteinfluss reduzierte körpereigene DNS-Repair-Kapazität der Hautzellen wieder auf das physiologisch benötigte Mass zu bringen und auf diese Weise dem Auftreten von bleibenden Zellschäden vorzubeugen und den lichtbedingten Alterungsprozess der Haut zu verlangsamen.

Mit den bisher bekannten Mitteln wird dies nicht erreicht:

a) Die in den „Chemical Abstracts", Bd. 87, Nr. 18, 31. Oktober 1977 (Columbus, Ohio, US), S. 315, column 1, abstract 141124b, beschriebenen Packungen enthalten Extrakte aus *Candida utilis* (Futterhefe), die im Vergleich zu konventionellen Hefen stabiler sind. Derartige Hefeextrakte sind reich an Vitaminen B und dienen seit langem in der Kosmetik zu Behandlung unreiner Haut. Irgendwelche Einflüsse auf die DNS-Repair-Kapazität sind nicht beschrieben und auch nicht zu erwarten.

b) Ferner sind Präparate bekannt („Chemical Abstracts", Bd. 88, Nr. 16, 17. April 1978 [Columbus, Ohio, US], S. 334, column 2, abstract 110537e), die Nährböden enthalten, wie sie zur Anzüchtung von Laktobazillen verwendent werden. Diese Nährböden enthalten Puffersubstanzen zum Abfangen von Milchsäure, die bei der Züchtung von Laktobazillen entsteht und hemmend auf deren Wachstum wirken würde. In den hier beschriebenen Präparaten sollen die Nährböden jedoch nicht zur Züchtung von Laktobazillen verwendet werden, sondern vielmehr abnormale Wirkungen von Milchsäure im Muskel vermeiden helfen, also als Milchsäurefänger dienen. Eine beeinflussung des DNS-Repair-Prozesses ist weder durch Nährböden noch durch die anderen Inhaltsstoffe dieser bekannten Präparate, wie pulverisierter Tee, Lecithin, zu erreichen und wird auch nicht beansprucht.

c) In der FR-A Nr. 1096421 ist ein kosmetisches Produkt beschrieben, das einen Gehalt an lebenden Milchgärstoffen mit saurem Charakter aufweist (*Lactobacillus acidophilus*). Diese Bakterien bilden bekanntlich Milchsäure als Stoffwechselprodukt, so dass bei Anwendung dieses Mittels der physiologische Säureschutzmantel der Haut erhalten bzw. wiederhergestellt wird, was mit einer Beeinflussung des DNS-Repair-Prozesses jedoch nichts zu tun hat.

d) Aus der FR-A Nr. 1112301 ist ein kosmetisches Mittel bekannt, das Milchgärstoffe enthält, und zwar in lebender Form. Als Milchgärstoff wird Propionibakterium verwendet, zu dessen Stoffwechselprodukten bemerkenswerte Mengen Riboflavin gehören, das eine günstige Hautwirkung besitzt. Riboflavin ist Vitamin $B_2$, d.h., dieses kosmetische Produkt zeichnet sich durch eine Vitamin-B-Wirkung auf die Haut aus, was, wie schon unter a erwähnt, bei der Behandlung unreiner Haut günstig ist; keinesfalls wird hierdurch aber der DNS-Repair-Prozess der Haut beeinflusst.

e) Das in US-A Nr. 4057627 beschriebene Erzeugnis ist ein Aknepräparat für die orale Anwendung, wobei inaktiviertes *Corynebacterium acnes* als immunisierendes Mittel gegen Akne verwendet wird. Es handelt sich also um einen oral zu verwendenden Impfstoff zur Aknebehandlung, so dass auch dieses Mittel nicht geeignet ist, dem Auftreten von bleibenden Zellschäden vorzubeugen und den lichtbedingten Alterungsprozess der Haut zu verlangsamen.

Nach der Erfindung wird diese Aufgabe dadurch gelöst, dass kosmetische Mittel als Wirkstoffkomplex inaktivierte Kulturen von Bakterien der Gattung *Bifido*-Bakterium oder dieser Gattung verwandte Bakterien enthalten. Ein derartiger, lokal zugeführter Wirkstoff ist imstande, die angestrebte Erhöhung der durch Lichteinwirkung reduzierten körpereigenen DNS-Repair-Kapazität der Hautzellen zu bewirken.

Durch die Anwendung dieser Mittel kann prophylaktisch dem Auftreten bleibender Zellschäden entgegengewirkt und der lichtbedingte Alterungsprozess der Haut verlangsamt werden, was diesen Mitteln vom kosmetischen Blickpunkt aus einen hohen praktischen Gebrauchswert verleiht.

Der den Repair-Prozess fördernde erfindungswesentliche Wirkstoffkomplex besteht aus inaktivierten Bakterien der Gattung *Bifido*-Bakterium, wie der Spezies *Bifidobakterium longum* (Reuter). Diese enthalten die Cytoplasma-Fraktion mit Enzymen, wie Milchsäuredehydrogenase, Phosphatasen, Phosphoketolasen und Transaldolasen, ausserdem Murein und Polysaccharide.

Die inaktivierten Zellen können z.B. erhalten werden, indem man anaerob gewonnene Oberflächenkulturen der *Bifido*-Bakterien mit physiologischer kochsalzlösung abspült, zentrifugiert, erneut in physiologischer Kochsalzlösung aufnimmt und unter Kühlung im Eisbad auf 0° C mit Ultraschall (20 kHz) bis zur völligen Abtötung aller vermehrungsfähigen Organismen behandelt. Dazu ist pro 100 ml Lösung eine Zeit von etwa 20 min erforderlich. Zur Inaktivierung der Zellen lassen sich auch andere, in der Literatur beschriebene mechanische Arbeitsmethoden anwenden, wie sie u. a. aus J. R. Norris und D. W. Ribbons „Methods in Microbiology", vol. 5 B, S. 1 bis 54 (1971), Verlag Academic Press London und New York, bekannt sind.

Es können aber auch der Gattung *Bifido*-Bakterium verwandte Bakterien als Ausgangsmaterial benutzt werden, wie sie z.B. in Bergeys „Manual of Determinative Bacteriology", 8. Auflage (1975), unter *Actinomycetaceae*, *Propionibacteriaceae*, *Lactobacillaceae* und coryneforme Bakterien genannt werden (vgl. dort u. a. die Seiten 576, 599, 633 und 659 bis 660).

Anhand von umfangreichen *in vitro*- und *in vivo*- Versuchsreihen wurde nachgewiesen, dass die natürliche DNS-Repair-Rate durch exogene Zufuhr des Wirkstoffkomplexes — eingearbeitet in kosmetische Mittel — erheblich gesteigert werden kann. In einzelnen Testreihen wurden sogar Steigerungsraten von mehr als 100% erzielt.

Die repair-fördernde Wirkung des erfindungswesentlichen Wirkstoffkomplexes konnte mit folgenden Versuchsreihen nachgewiesen werden.

I. In vitro- *Testreihen an*
Mausmilzzellen,
menschlichen Lymphosyten, und
menschlischen Fibroblasten

In analogen Experimenten wurde das jeweilige Zellmaterial in einem Lösungsmittel suspendiert, mit dem zu testenden Wirkstoffkomplex versetzt und mit einer UV-Lampe bei 254 nm bestrahlt. Als Bezug diente eine gleich behandelte Kontrollreihe, die aber ohne Wirkstoffzusatz blieb.

Um die programmierte DNS-Replikation, die sogenannte semikonservative Synthese zu unterdrücken, wurde als Hemmstoff Hydroxyharnstoff zugesetzt. Die jetzt nur noch ablaufende, sogenannte unprogrammierte Synthese ist ein direktes Mass für den Exzisions-Repair-Prozess.

Nach der Bestrahlung wurde jedem Ansatz tritiiertes $H^3$-Thymidin zugesetzt. Dieses radioaktiv markierte Thymidin wurde im Verlauf des Repair-Prozesses gegen das fehlerhafte Thymidindimere ausgetauscht. Die anhand der Markierung messbare Einbaurate des $H^3$-Thymidins in die DNS ist ein Mass für die Repair-Rate.

Als Ergebnis wurde festgestellt: In allen Fällen zeigte der Wirkstoffkomplex gegenüber den Kontrollen eine hochsignifikante Erhöhung der Einbaurate des $H^3$-Thymidins.

II. In vivo- *Testreihe an Ratten*

Den Tieren wurden auf rasierten Feldern des Rückens die zu testenden Substanzen aufgetragen: Wirkstoffkomplex und daneben kosmetische Mittel mit einem Gehalt von 5 bzw. 10% Wirkstoffkomplex. Zur Relativierung der Ergebnisse kamen jeweils entsprochenden Blindsubstanzen zur Anwendung. Als absoluter Bezug für Wirk- und Blindsubstanzen dienten damit behandelte, aber unbestrahlte Areale der Ratte.

Vor der Bestrahlung wurden einer Testgruppe von 10 Tieren im Abstand von 4 h jeweils 50 mg Test- und Blindsubstanzen auf die dazu vorgesehenen Areale aufgetragen. 72 h nach der ersten Substanzapplikation erfolgte die UV-Bestrahlung. Eine Kontrollgruppe von 10 Tieren blieb unbestrahlt. Nach der Bestrahlung wurde den Tieren der Versuchs- und Kontrollgruppe eine Lösung aus $H^3$-Thymidin und zur Unterdrückung der programmierten DNS-Synthese Hydroxyharnstoff injiziert. Die Tötung der Tiere aller Gruppen erfolgte 24 h nach der Bestrahlung in Äthernarkose. Auch hier ist die ermittelte $H^3$-Thymidin-Einbaurate ein Mass für die Repair-Aktivität der Testsubstanz.

Als Ergebnis wurde festgestellt: Der Wirkstoffkomplex zeigte eine statistisch signifikante Erhöhung der Repair-Kapazität gegenüber den Kontrollen. Ein weiteres wichtiges Ergebnis war, dass das kosmetische Mittel mit 10% des repair-fördernden Wirkstoffkomplexes eine vierfach höhere, und das kosmetische Mittel mit 5% eine zweifach höhere Repair-Aktivität als der Wirkstoffkomplex selbst zeigte.

Die nachfolgenden Rezepturen sind Ausführungsbeispiele der Erfindung.

*Beispiel 1*

*Creme O/W mit 10% eines nach den obigen Angaben hergestellten, repair-fördernden Wirkstoffkomplexes*

Es wurden folgende Ingredienzien verwendet:

| | | |
|---|---|---|
| a) | Cetylstearylalkohol | 9,0% |
| | Natriumcetylstearylsulfat | 1,0% |
| | Ölsäuredecylester | 10,0% |
| | Wollfett | 2,0% |
| | Triglycerid von fraktionierten Kokosölfettsäuren | 5,0% |
| | Konservierungsmittel | q.s. |
| b) | destilliertes Wasser | 57,7% |
| | 70%ige Lösung von Sorbit | 5,0% |
| c) | repair-fördernder Wirkstoffkomplex | 10,0% |

Die Zubereitung des Mittels erfolgt in der Weise, dass zunächst die Ingredienzienkombination a geschmolzen und auf ca. 70° C erwärmt und in die erhaltene Schmelze die unter b angeführte, auf 70° C erwärmte Lösung eingerührt wird. Das Ganze wird weitergerührt, bis eine Abkühlung der Creme auf ca. 35° C eingetreten ist. Danach wird die Komponente c eingerührt, bis sie gleichmässig verteilt ist.

*Beispiel 2*

*Creme W/O mit 10% eines nach den obigen Angaben hergestellten, repair-fördernden Wirkstoffkomplexes*

Es wurden folgende Ingredienzien verwendet:

| | | |
|---|---|---|
| a) | Gemisch aus hochmolekularen Estern, vorwiegend Mischestern aus Pentaerythritfettsäureester und Zitronensäurealkoholester und mineralischen Fetten | 20,0% |
| | Ölsäuredecylester | 5,0% |
| | Pflanzenöl | 5,0% |
| | weisse Vaseline | 5,0% |
| | Konservierungsmittel | q.s. |
| b) | destilliertes Wasser | 55,0% |
| c) | repair-fördernder Wirkstoffkomplex | 10,0% |

Die Zubereitung des Mittels erfolgt nach der in Beispiel 1 angegebenen Methode.

*Beispiel 3*

*Gel mit repair-förderndem Wirkstoffkomplex*

| | | |
|---|---|---|
| a) | Wasser, destilliert, konserviert | 50,0% |
| | Carbopol 940 (Polyacrylsäure) | 0,5% |
| | Phenonip (Konservierungsmittel) | 0,3% |
| b) | Wasser, destilliert, konserviert | 28,2% |
| | Triäthanolamin | 1,0% |
| c) | repair-fördernder Wirkstoffkomplex | 20,0% |

Die Zubereitung des Mittels erfolgt in der Weise, dass die Ingredienzienkombination a durch schnelles Rühren dispergiert wird. Danach wird die unter b angeführte Lösung und anschliessend die Komponente c eingerührt.

*Beispiel 4*

*Lotion mit repair-fördernden Wirkstoffkomplex*

| | | |
|---|---|---|
| a) | Äthanol 96 Vol.-% | 15,0% |
| b) | Wasser, destilliert, konserviert | 76,4% |
| | Zitronensäure | 0,3% |
| | Phenonip | 0,3% |
| | 1,2-Propylenglykol | 3,0% |
| c) | repair-fördernder Wirkstoffkomplex | 5,0% |

Die Zubereitung des Mittels erfolgt in der Weise, dass aus den Ingredienzien unter b eine Lösung hergestellt wird. Danach wird zuerst die Komponente a und anschliessend die Komponente c eingerührt.

## Patentansprüche

1. Kosmetische Mittel, dadurch gekennzeichnet, dass sie als einen den DNS-Repair-Prozess der Hautzellen fördernden Wirkstoffkomplex inaktivierte Kulturen von Bakterien der Gattung *Bifido*-Bakterium oder dieser Gattung verwandte Bakterien enthalten.

2. Kosmetische Mittel nach Anspruch 1, dadurch gekennzeichnet, dass der Wirkstoffkomplex aus inaktivierten Kulturen folgender, der Gattung *Bifido*-Bakterium verwandter Bakterien besteht: *Actinomycetaceae, Propionibacteriaceae, Lactobacillaceae* und coryneforme Bakterien.

## Claims

1. Cosmetic preparations, characterized in that for promoting the DNA-repair process of skin cells they contain, as an active substance, complex inactivated cultures of bacteria of the genus *Bifidobacterium* or of bacteria which are related to this genus.

2. Cosmetic preparations according to claim 1, characterized in that the active substance complex consists of inactivated cultures of the following bacteria which are related to the genus *Bifidobacterium*: *Actinomycetaceae, Propionibacteriaceae, Lactobacillaceae* and coryneform bacteria.

## Revendications

1. Produits cosmétiques, caractérisés par le fait qu'ils contiennent, comme complexe de substances actives favorisant le processus de réparation de l'ADN des cellules de la peau, des cultures inactivées de bactéries du genre *Bifidobacterium* ou de bactéries apparentées à ce genre.

2. Produits cosmétiques selon la revendication 1, caractérisés par le fait que le complexe de substances actives est constitué de cultures inactivées des bactéries apparentées au genre *Bifidobacterium* suivantes: *Actinomycetaceae, Propionibacteriaceae, Lactobacillaceae* et corynébactéries.